# EUROPEAN PATENT SPECIFICATION

(11) **EP 2 699 275 B1**
(45) Date of publication and mention of the grant of the patent: **07.03.2018**
(21) Application number: 12716172.7
(22) Date of filing: 17.04.2012
(51) Int. Cl.: A61L 27/04, A61L 27/50, A61L 27/56, A61L 31/02, A61L 31/14, A61F 2/30, B23K 20/02, B23K 20/16, B23K 20/233, B23K 35/32, B23K 103/18

(54) **METHOD FOR BONDING A TANTALUM STRUCTURE TO A COBALT-ALLOY SUBSTRATE**
VERFAHREN ZUM BONDEN EINER TANTALSTRUKTUR AN EIN KOBALTKUPFERSUBSTRAT
PROCÉDÉ DE LIAISON D'UNE STRUCTURE DE TANTALE À UN SUBSTRAT D'ALLIAGE DE COBALT

(30) Priority: 22.04.2011 US 201113092169
(43) Date of publication of application: 26.02.2014
(73) Proprietor: Zimmer, Inc., Warsaw IN 46580 (US)
(72) Inventor: HIPPENSTEEL, Gregory M., South Whitley, IN 46787 (US); PEEK, Lawrence F., Warsaw, IN 46582 (US); ANDERSON, Jeffrey P., Warsaw, IN 46582 (US); GORHE, Devendra, Warsaw, IN 46582 (US); ALLEN, Steve M., Winona Lake, IN 46590 (US); PANCHISON, Clarence M., Warsaw, IN 46582 (US); MILLER, David M., Warsaw, IN 46580 (US); SCRAFTON, Joel G., Syracuse, IN 46567 (US); HARMON, Casey, Warsaw, IN 46580 (US)
(74) Representative: Mays, Julie
(86) International application number: PCT/US2012/033898
(87) International publication number: WO 2012/145292

(56) References cited:
- EP-A2- 2 047 937
- US-A1- 2008 050 699
- GIULIO MACCAURO AND AL.: "An overview about biomedical applications of micron and nano size Tantalum", RECENT PATENTS ON BIOTECHNOLOGY, vol. 3, no. 3, 2009, pages 157-165, XP002680576,

## Description

### FIELD OF THE INVENTION

This invention relates generally to orthopedic implants, and more particularly relates to a method for bonding a porous tantalum structure to cobalt or a cobalt-alloy orthopedic implant.

### BACKGROUND OF THE INVENTION

Orthopedic implants are often utilized to help their recipients recover from injury or disease. To promote quick recovery, orthopedic implants are designed to cooperate with the body's natural inclination to heal itself. Some orthopedic implants are designed to foster osseointegration. Osseointegration is the integration of living bone within a man-made material, usually a porous structure. Cells in the recipient form new bone within the pores of the porous structure. Thus, the porous structure and the bone tissue become intermingled as the bone grows into the pores. Accordingly, orthopedic implants can include a porous surface to enhance fixation between the orthopedic implant and adjacent tissue. Of course, the faster the surrounding tissue grows into the porous surface, the sooner the patient can begin to resume normal activities. However, the manufacture of the orthopedic implants with porous structures is not without difficulty.

Orthopedic implants are usually made from various metals. One difficulty encountered during manufacturing is bonding separate components, each made of a different metal, together. For example, cobalt is a popular metal used to make orthopedic implants, and other popular metals include alloys of cobalt with other metals, such as chromium. The porous structure can be made from an entirely different metal, such as tantalum. In this case, bonding the porous metal to the orthopedic implant involves bonding tantalum to cobalt or to cobalt-chromium alloys. Bonding these two metals together has proved to be particularly problematic.

EP 2047937 A discloses a method for bonding a porous tantalum structure to a substrate, comprising applying heat and pressure to a substrate and an interlayer for a time sufficient to achieve solid-state diffusion.

G. Maccauro et al., Recent Patents on Biotechnology 3(3), 157-165 (2009) discloses a study of the properties of porous tantalum and the bonding technology between porous tantalum and cobalt or cobalt-chromium alloys.

Thus, there is a need for an improved method of bonding of porous structures, specifically tantalum, to cobalt and cobalt-alloy implants such that the bond has sufficient strength while the corrosion resistance of the metals in the resulting implant are maintained.

### SUMMARY OF THE INVENTION

The present invention provides a method for bonding a porous tantalum structure to a substrate as defined in claim 1. Also described is a method comprising providing (i) a substrate comprising cobalt or a cobalt-chromium alloy; (ii) an interlayer consisting essentially of at least one of hafnium, manganese, niobium, palladium, zirconium, titanium, or alloys or combinations thereof; and (iii) a porous tantalum structure, and applying heat and pressure for a time sufficient to achieve solid-state diffusion between the substrate and the interlayer and solid-state diffusion between the interlayer and the porous tantalum structure.

Also described is a method for bonding a porous tantalum structure to a substrate. The method comprises positioning a compressible interlayer between a porous tantalum structure and a substrate comprising cobalt or cobalt-chromium to form an assembly wherein the compressible interlayer consists essentially of a metal or alloy that exhibits solid solubility with the porous tantalum structure and the substrate. Heat and pressure are applied to the assembly for a time sufficient to achieve solid-state diffusion between the substrate and the compressible interlayer and solid state diffusion between the compressible interlayer and the porous tantalum structure.

Also described is a method for bonding a porous tantalum structure to a substrate. The method includes providing a porous tantalum structure in a first configuration and providing a substrate comprising cobalt or cobalt-chromium. A porous interlayer is applied to a surface of the porous tantalum structure to form a subassembly wherein the porous interlayer comprises a metal or alloy that is soluble in the solid state with both the porous tantalum structure and the substrate. The subassembly is bent into a second configuration and a surface of the substrate is brought into contact with the interlayer to create an assembly. Heat and pressure are applied to the assembly for a time sufficient to achieve solid-state diffusion between the substrate and the interlayer and solid state diffusion between the interlayer and the porous tantalum structure.

Also described is an assembly for forming a medical implant. The assembly comprises a porous tantalum structure and a substrate comprising cobalt or cobalt-chromium alloy. The assembly also includes a compressible interlayer positioned between the porous tantalum structure and the substrate, wherein the compressible interlayer consists essentially of a metal or alloy that exhibits solid solubility with the porous tantalum structure and the substrate.

Also described is a medical implant comprising a porous tantalum structure and a substrate made of cobalt or cobalt-chromium alloy. The implant further includes a compressed interlayer between a surface of the porous tantalum structure and a surface of the substrate. The compressed interlayer consists essentially of a metal or alloy that exhibits solid solubility with the porous tantalum structure and the substrate.

### BRIEF DESCRIPTION OF THE DRAWINGS

The accompanying drawings, which are incorporated in and constitute a part of this specification, illustrate certain embodiments of the invention and, together with a general description of the invention given above, and the detailed description given below, serve to explain the invention.
FIG. 1 depicts a cross-sectional view of an assembly comprising a porous tantalum structure, a pre-formed sheet interlayer, and a substrate;
FIG. 2 depicts a cross-sectional view of an assembly comprising a porous tantalum structure, a coating interlayer, and a substrate;
FIGS. 3 and 4 are photomicrographs corresponding to the embodiments of FIGS. 1 and 2, respectively, following heating and pressing the assembly to bond the porous tantalum structure to the interlayer and the interlayer to the substrate;
FIG. 5 is a perspective view of an exemplary embodiment of a cobalt-chromium femoral implant that can have a porous tantalum structure bond thereto in accordance with the methods of the present disclosure;
FIG. 6 is an exploded perspective view of one embodiment of a femoral implant construct of the present disclosure including a porous tantalum structure and a substrate;
FIG. 7 is a planar view of the porous tantalum structure of FIG. 6 shown in a substantially flat configuration;
FIG. 8 is perspective view of the femoral implant construct of FIG. 6;
FIG. 9 is a photomicrograph showing a porous tantalum structure having a coated interlayer applied thereto;
FIG. 10 is a photomicrograph showing a compressed interlayer bonded to a tantalum structure and to a substrate;
FIG. 11 is a laser holography image of a construct made using a solid interlayer; and
FIG. 12 is a laser holography image of a construct made using a compressible interlayer.

### DETAILED DESCRIPTION

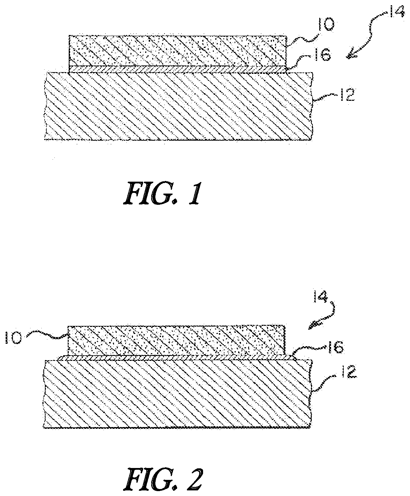

With reference to FIGS. 1 and 2, a method for bonding a porous tantalum structure 10 to a substrate 12 can begin by constructing an assembly 14 comprising an interlayer 16 placed on the surface of the substrate 12 and the porous tantalum structure 10 placed onto the interlayer 16. It will be appreciated that the assembly 14 can be constructed by placing the individual components 10, 12, 16 together in any order that results in the interlayer 16 positioned between and in contact with the substrate 12, and the porous tantalum structure 10, as shown in FIGS. 1 and 2. In other words, the placement order is not limited to those orders described herein.

The porous tantalum structure 10 can be TRABECULAR METAL®, available from Zimmer Inc., Warsaw, Ind. The porous tantalum structure 10 can be configured to facilitate osseointegration. The porous tantalum structure 10 can have a pore size, pore continuity, and other features for facilitating bone tissue growth into the pores, as is known in the art.

The substrate 12 can be a cast or a wrought cobalt or cobalt chromium alloy fabricated in a shape according to the requirements for the specific orthopedic application. For example, the substrate 12 can be cast of cobalt in the shape of a total hip replacement implant. Other implants can include implants for the ankle, elbow, shoulder, knee, wrist, finger, and toe joints or other portions of the body that can benefit from a substrate 12 having a porous tantalum structure 10 bonded thereto.

With no intent to be bound by theory, tantalum and cobalt metals are not readily soluble, that is, the documented solid solubility of tantalum into cobalt is insufficient to form the necessary bond strength demanded by applications within the human body. In fact, certain stoichiometries of tantalum with cobalt can prevent solid state diffusion of tantalum into cobalt and vice versa. Therefore, the interlayer 16 can comprise a metal that readily forms solid solutions with both tantalum and cobalt or cobalt-chromium alloys. The interlayer 16 can be any one or an alloy of metals, such as, hafnium, manganese, niobium, palladium, zirconium, titanium, or other metals or alloys that exhibit solid solubility with tantalum at temperatures less than the melting temperature of the substrate 12, the interlayer 16, or the porous tantalum structure 10.

The assembly 14, as shown in FIGS. 1 and 2, can be put together by applying the interlayer 16 to the substrate 12. The interlayer 16 can require pre-shaping to improve the contact area between the surface of the substrate 12 and the surface of interlayer 16 prior to applying the interlayer 16 to the substrate 12. Alternatively, the interlayer 16 can be press formed onto the substrate 12 such that the interlayer 16 conforms to the surface of the substrate 12. The surfaces of all components 10, 12, 16 can be cleaned prior to assembly 14 to reduce corrosion and improve solid-state diffusion bonding.

With continued reference to FIGS. 1 and 2, following application of the interlayer 16 to the substrate 12, the porous tantalum structure 10 can be placed on the interlayer 16 to form the assembly 14. Similar to pre-shaping the interlayer 16 to conform to the substrate 12, the porous tantalum structure 10 can be formed in a shape to maximize surface-to-surface contact to facilitate solid-state diffusion with the interlayer 16.

Heat and pressure can be applied to the assembly 14 sufficient for solid-state diffusion to take place between the substrate 12 and the interlayer 16 and between the interlayer 16 and the porous tantalum structure 10. Solid-state diffusion is the movement and transport of atoms in solid phases. Solid-state diffusion bonding can form a monolithic joint through formation of bonds at an atomic level due to transport of atoms between two or more metal surfaces. Heat and pressure can be supplied to the assembly 14 with a variety of methods known in the art. For example, the assembly 14 can be heated electrically, radiantly, optically, by induction, by combustion, by microwave, or other means known in the art. Pressure can be applied mechanically by clamping the assembly 14 together prior to insertion of the assembly 14 into a furnace, or pressure can be applied via a hot pressing system capable of applying pressure once the assembly 14 reaches a target temperature. Furthermore, hot pressing can include hot isostatic pressing, also known in the art.

Referring now to FIG. 1, the interlayer 16 can be a preformed sheet of commercially pure titanium at least (about 0.016 inches) about 0.04064 centimeter thick. The pre-formed sheet of commercially pure titanium can be at least about (0.020 inches) about 0.0508 centimeter thick for improved bond strength. The interlayer 16 can be positioned directly beneath the porous tantalum structure 10. In other words, it is not necessary to cover the entire substrate 12 with the interlayer 16 to bond the porous tantalum structure 10 at a single location. Furthermore, the corrosion resistance and the strength of the substrate 12 are not negatively impacted if the porous tantalum structure 10 touches those areas not covered by the interlayer 16 during heating. Thus, the porous tantalum structure 10 can be bonded to multiple separate areas on the surface of the substrate 12 with multiple separate areas of interlayer 16. The position of the porous tantalum structure 10 can be dictated by the patient's physiological requirements.

The assembly 14 can be clamped together by applying a pressure of at least approximately (200 pounds per square inch (psi)) approximately 1.38 MPa. However, pressures greater than approximately 1.38 MPa (200 psi) can be applied up to the compressive yield strength of the any of the substrate 12, the interlayer 16, or the porous tantalum structure 10. Ordinarily, the porous tantalum structure 10 can have the lowest compressive yield strength, for example, 40.0 MPa (5,800 psi) for TRABECULAR METAL®.

The clamped assembly 14 can then be heated to at least about 540 ° C (about 1004 degree Fahrenheit) in vacuum or in another sub-atmospheric pressure of an inert atmosphere. In any case, the clamped assembly 14 can be heated to less than the melting temperature of any of the components 10, 12, 16 and, in most cases, can be at least about 800°C (about 1472 degree Fahrenheit) but less than about 1000°C (about 1832 degree Fahrenheit) in vacuum. The higher the temperature, the less time it can take to achieve solid-state diffusion bonding. The time required to achieve solid-state diffusion bonding can be as little as less than 1 hour to as long as 48 hours and can depend on the metals involved, the temperatures, atmosphere, and the pressures applied.

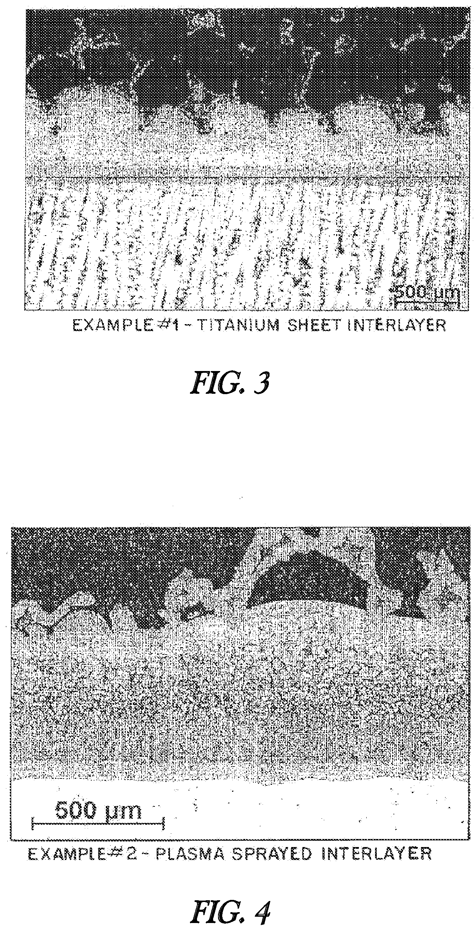

Once heated to temperature, and after a time sufficient to achieve solid-state diffusion between the porous tantalum structure 10 and the interlayer 16 and between the interlayer 16 and the substrate 12, a construct can be formed. The construct can comprise the substrate 12 bonded to the interlayer 16 and the interlayer 16 bonded to the porous tantalum structure 10. FIG. 3 is a photomicrograph of a portion of the construct formed according to one embodiment of the method, described above, with a porous tantalum structure 10 (top) bonded to a titanium sheet interlayer 16 (middle) bonded to a cobalt-chromium substrate 12 (bottom).

With reference now to FIG. 2, the interlayer 16 can be a coating applied to the surface by, for example, thermal spray, plasma spray, electron beam deposition, laser deposition, cold spray, or other method of forming the coatings on a substrate 12. The coating interlayer 16 can be applied via vacuum plasma spraying. The substrate 12 can be masked and then grit blasted to prepare the surface of the substrate 12 for vacuum plasma spraying. The substrate 12 can be masked and then grit blasted with alumina (aluminum oxide) grit for increased corrosion resistance of the construct subsequent to bonding with the interlayer 16. The coating interlayer 16 can comprise titanium sprayed to a thickness of at least about 0.010 inches (about 0.0254 centimeter) thick. For increased bond strength, the titanium coating interlayer 16 can be at least about 0.020 inches (about 0.0508 centimeter) thick. When vacuum plasma spraying is used, a porosity level can be between about 20% and about 40% for ease of vacuum plasma spray processing while maintaining sufficient corrosion resistance. The porosity can be at least about 5%, such as at least about 20%, at least about 30% or at least about 40%. In an example, the porosity can be between about 30% and about 40%. A plasma sprayed interlayer can include adjoining metal particles or ligands defining pores therebetween. The porosity of the interlayer can allow for compressibility of the interlayer, which compressibility can be advantageous and desired in some applications. FIG. 4 is a photomicrograph of a portion of a construct formed according to one embodiment of the method described above, showing a portion of a construct comprising a porous tantalum structure 10 (top) bonded to a titanium vacuum plasma sprayed interlayer 16 (middle) bonded to a cobalt-chromium substrate 12 (bottom).

Coated interlayer 16 can be coated on either the porous tantalum structure 10 or the substrate 12 by any of the coating processes disclosed above. For example, the coated interlayer 16 can be applied by plasma spraying. When the surface of substrate 12 is geometrically complex, it can be difficult to form a coated interlayer of uniform thickness on the surface of the substrate. A coated interlayer of non-uniform thickness can result in undesired incongruency between the surfaces of the substrate and tantalum porous structure. It also can result in incomplete bonding of the tantalum porous structure to the substrate and undesired surface deviations.

As used herein a "geometrically complex" surface of a substrate is a surface that is other than a simple continuous flat surface. Such geometrically complex surfaces can include, but are not limited to, surfaces that include two or more flat sections that project at an angle with respect to each other, surfaces that include multiple flat sections wherein the flat sections project at angles with respect to adjacent sections, non-flat surfaces, rounded surfaces, concave surfaces, convex surfaces, and combinations thereof. When it is difficult to coat the interlayer on the surface of the substrate because of the surface's geometry, or for some other reason, the interlayer can be coated onto a surface of the porous tantalum structure instead of a surface of the substrate.

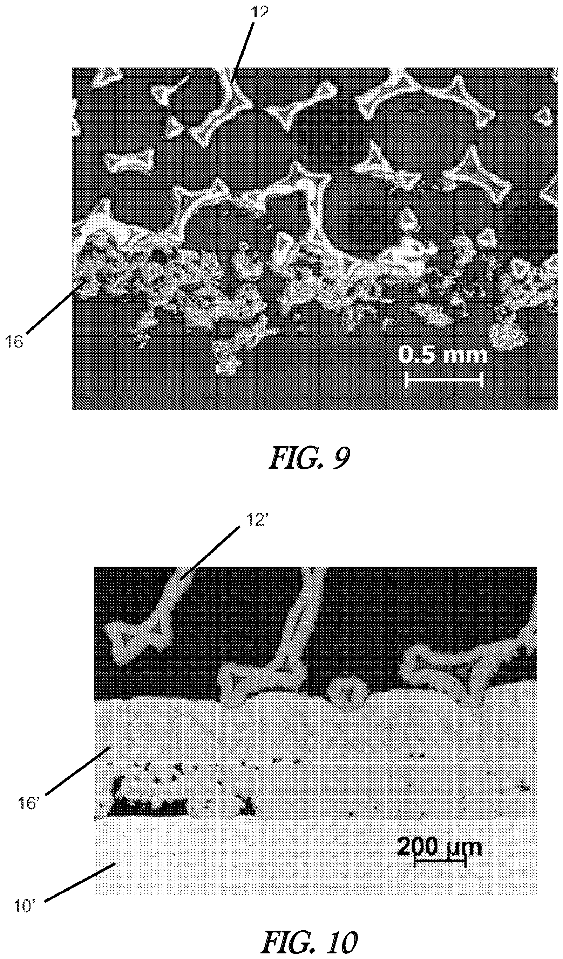

One concern with applying a coated interlayer 16 to a surface of the porous tantalum structure 12 can be that the potential for coated interlayer 16 to occlude or block the pores of porous tantalum structure 12. For example, during the plasma spraying process, the metal which forms interlayer 16 can be formed into liquid particles, which particles can be applied to porous tantalum structure 12. It was thought that such liquid particles can enter the pores of porous tantalum structure 12 where the particles can solidify and occluded the pores of tantalum structure 12. However, in accordance with the methods disclosed herein, coated interlayer 16 can be applied or coated onto porous tantalum structure 12 without causing significant pore occlusion. Fig. 9 is a microphotograph of a portion of a tantalum structure 12 having a plasma sprayed interlayer 16 coated thereon. As shown in Fig. 9, the interlayer 16 does not significantly occlude the porous tantalum structure 12.

A construct comprising a porous tantalum structure 10 of TRABECULAR METAL® bonded to a titanium interlayer 16 bonded to a cobalt-chromium substrate 12 can be characterized by tensile strength testing. Nearly all failure separations occurred in the porous tantalum structure 10. Tensile stresses measured at separation on constructs formed according to the previously described embodiments were routinely above 20 MPa (2,900 psi).

Heating and applying pressure can include multiple heating and pressurizing processes. For example, the porous tantalum structure 10 can be assembled with the interlayer 16 and bonded thereto, according to one embodiment of the method, to form a subassembly. That subassembly can then be bonded to the substrate 12 according to another embodiment of the method. The reverse procedure can also be used. That is, the interlayer 16 can be bonded to the substrate 12 to form a subassembly with subsequent bonding of the porous tantalum structure 10 to the interlayer portion of the subassembly. Therefore, embodiments of the method can account for different diffusion coefficients between the components 10, 12, 16 which can allow for more consistent, higher strength bonds between the substrate 12 and interlayer 16 and between the interlayer 16 and the porous tantalum structure 10. By way of further example and not limitation, diffusion bonding of a titanium interlayer 16 to a cobalt-chromium substrate 12 at an elevated temperature and pressure can take longer than diffusion bonding of the titanium interlayer 16 to a porous tantalum structure 10 at similar pressures and temperatures. Thus, by diffusion bonding the titanium interlayer 16 to the cobalt-chromium substrate 12 to form a subassembly and then diffusion bonding the porous tantalum structure 10 to the subassembly, a diffusion bond depth between the titanium interlayer 16 and the cobalt-chromium substrate 12 can be substantially the same as a diffusion bond depth between the titanium interlayer 16 and the porous tantalum structure 10. In contrast, if the porous tantalum structure 10, the titanium interlayer 16, and the cobalt-chromium substrate 12 are bonded with a single application of heat and pressure, the diffusion bond depths between the titanium interlayer 16 and the porous tantalum structure 10 and between the titanium interlayer 16 and the cobalt-chromium substrate 12 can be different.

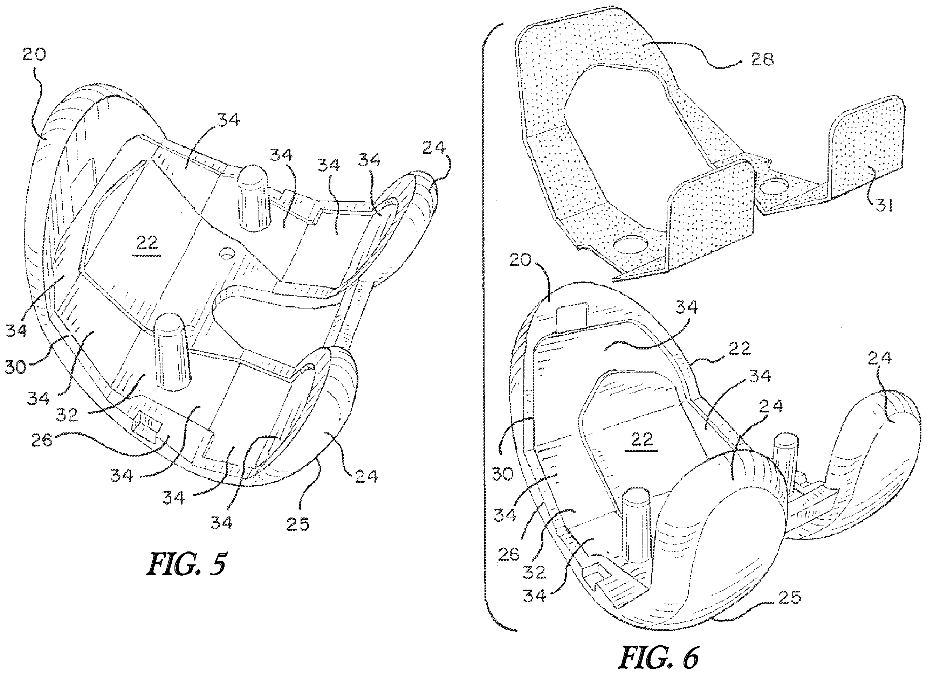

Figs. 5 and 6 illustrate one exemplary embodiment of a substrate having a geometrically complex surface. In particular, the illustrated substrate can be a cobalt-chromium femoral knee implant 20. Although the following is described with reference to femoral implant 20, the substrate having a geometrically complex surface can be any cobalt or cobalt-chromium substrate, such as those used as ankle, shoulder, wrist, finger, toe, hip and elbow implants. Femoral knee implant 20 can include a main body portion 22 and a pair of condyle members 24 extending therefrom. Implant 20 can also include a bottom surface 25 for articulating against a tibial implant and a top surface 26 which can be configured to interface with the femur. Generally, a porous tantalum structure 28 (Fig. 6) can be bonded by an interlayer (not shown) to top surface 26. Top surface 26 can include a recessed generally U-shaped section 30 that can be configured to receive the similarly shaped porous tantalum structure 28. U-shaped section 30 can include a geometrically complex surface 32 that has nine flat sections 34 wherein each flat section can extend at an angle relative to adjacent flat sections.

In an example of a process of bonding porous tantalum structure 28 to surface 32, the interlayer can be coated, for example by plasma spray, to either surface 32 of implant 20 or surface 31 of porous tantalum structure 28. After the coated interlayer has been applied, any of the diffusion bonding processes described herein can then be used to bond porous tantalum structure 28 and implant 20 to the interlayer.

As discussed above, it can be difficult to coat a uniform interlayer having a consistent thickness to geometrically complex surface 32. In such instances, the interlayer can be coated, for example by plasma spraying, onto surface 31 of porous tantalum structure 28.

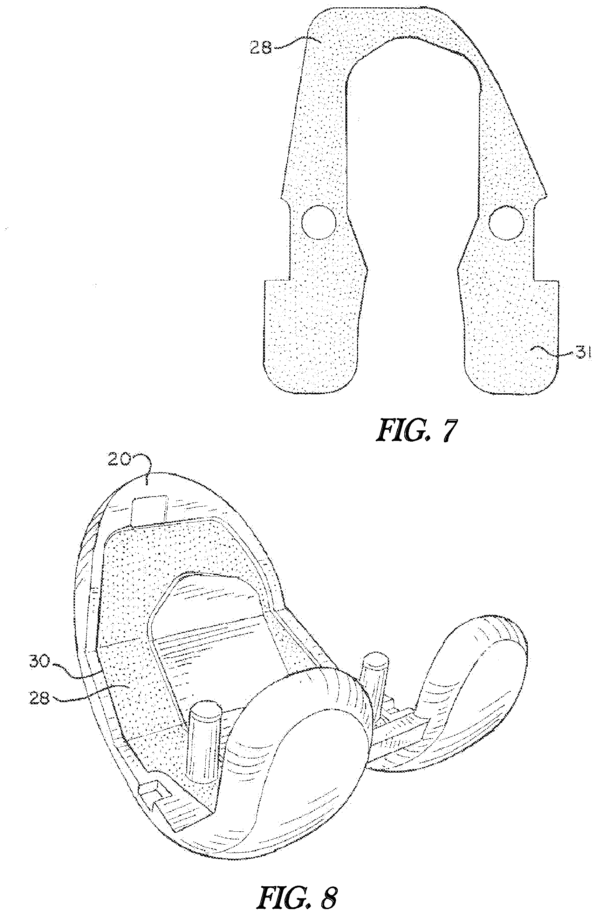

Referring to Fig. 7, porous tantalum structure 28 can have a first or initial configuration, such as the substantially flat configuration shown in this figure. While in this substantially flat configuration, the interlayer (not shown) can be coated onto surface 31 of porous tantalum structure 28 wherein surface 31 can be the surface bonded to surface 32 of implant 20 via the interlayer. The interlayer can be coated onto surface 31 of porous tantalum structure 28 by, for example, plasma spraying. Coating the interlayer onto porous tantalum structure 28 while structure 28 is in the substantially flat configuration can make it easier to achieve an interlayer with a substantially uniform thickness.

After the interlayer has been coated onto surface 31 of porous tantalum structure 28, structure 28 can then be bent into a second configuration. In the embodiment illustrated in Fig. 6, porous tantalum structure 28 can be bent so that the shape of structure 28 can be substantially congruent to recessed section 30 and geometrically complex surface 32 of implant 20. Porous tantalum structure 28 can be bent so that surface 31 has nine substantially flat sections corresponding to the nine substantially flat sections 34 of surface 32. Porous tantalum structure 28 can be placed in recessed U-shaped section 30 so that the interlayer coated on surface 31 of structure 28 can be placed in contact with surface 32 of implant 20. Any of the diffusion bonding processes described herein can then be used to bond porous tantalum structure 28 and implant 20 to the interlayer to form the construct illustrated in Fig. 8.

As discussed above, when an interlayer is porous, the porosity can allow the interlayer to be a compressible interlayer. For example, a plasma sprayed interlayer can include a porosity which allows the interlayer to be compressible. When sufficient pressure is placed on the porous interlayer, the pores of the interlayer can collapse, resulting in compression of the interlayer. The compressible interlayer can be compressed during the diffusion bonding process. In particular, during diffusion bonding, heat and pressure can be applied to the substrate, porous tantalum structure and the interlayer to bond the same together. The pressure applied during this bonding process can be sufficient to collapse the pores of the interlayer so as to compress the interlayer. Compression of the interlayer or portions thereof can result in the thickness of the interlayer or portion thereof being less than the thickness in the original uncompressed state. The interlayer can be uniformly compressed across the interlayer or can be non-uniformly compressed such that only certain areas or sections of the interlayer are compressed. Fig. 10 is a photomicrograph illustrating one embodiment of a construct shown after the diffusing bonding process. The construct includes a porous tantalum construct 12', a compressed interlayer 16' and a substrate 10'. As shown in this figure, the pores of compressed interlayer 16' can be collapsed.

Such a compressible interlayer can advantageously assist in providing a substantially complete bond between the substrate and tantalum porous structure across substantially all of the facing surfaces of the substrate and tantalum structure. In some examples, such as when the porous tantalum structure is bonded to a geometrically complex surface of a substrate, there can be deviations from the geometrical congruencies between the substrate and the porous tantalum structure. Such deviations can include deviations from parallelism, unintended curvature, and dimensional mismatch. When such deviations exist and the interlayer is substantially incompressible, for example when the interlayer is a substantially solid sheet, bonding quality between the tantalum porous structure and the substrate can be poor and unequal across the surfaces and the tantalum porous structure may not completely bond to the substrate. On the other hand, when such deviations exist and the interlayer is a compressible interlayer, the compression of the interlayer can compensate for such deviations, resulting in a relatively higher quality bond in which the bond between the porous tantalum structure and the substrate can be substantially complete.

### Example

A comparison was made to determine if there were any differences in the bonding between constructs formed by bonding porous tantalum structures to substrates with compressible interlayers and with incompressible interlayers. The porous tantalum structures used in this comparison are available from Zimmer, Inc., Warsaw, Ind. and sold under the trademark Trabecular Metal®. Additionally, the cobalt-chromium femoral knee implants used in this comparison are similar to those shown in Figs. 5 and 6 and are also available from Zimmer, Inc., Warsaw, Ind.

A solid, nonporous substantially incompressible interlayer sheet of titanium having a thickness of about (0.020 inches) 0.51 mm was employed in a diffusion bonding process to bond a porous tantalum structure having a thickness of about (0.045) 1.1 mm and a porosity of about 80% to the geometrically complex surface of a femoral implant. The bonding process included placing the sheet interlayer between the porous tantalum structure and the substrate and simultaneous bonding of the sheet interlayer to the substrate, and the porous tantalum to the sheet interlayer. The diffusion bonding process included about (1000 lbs) 6.89MPa of fixture pressure using a multi-piece compression tool, and bonding at 940°C (1725°F) for approximately one hour in a vacuum environment.

A porous compressible layer was used in a diffusion bonding process to bond a second porous tantalum structure having a thickness of (0.045 inches) 1.1 mm and a porosity of 80% to the geometrically complex surface of a second femoral implant. The bonding process included using a plasma sprayer available from Orchid Bio-Coat, Southfield, MI to plasma spray a titanium porous compressible interlayer onto the a surface of the second porous tantalum structure while the second porous tantalum structure was provided in a substantially flat configuration, such as the configuration shown in Fig. 7. The plasma sprayed interlayer had a thickness of approximately 0.063 cm (0.025 inches) and a porosity of approximately 30% to 40%. The substantially flat porous tantalum structure was then bent so that the coated surface of the tantalum structure substantially corresponded with the geometrically complex surface of the femoral implant. The interlayer on the coated surface of the porous tantalum structure was then placed in contact with the geometrically complex surface of the femoral implant and bonded thereto by diffusion bonding to form a second construct. The diffusion bonding process included about 6.89MPa (1000 lbs) of fixture pressure using a multi-piece compression tool, and bonding at 940°C (1725°F) for approximately one hour in a vacuum environment.

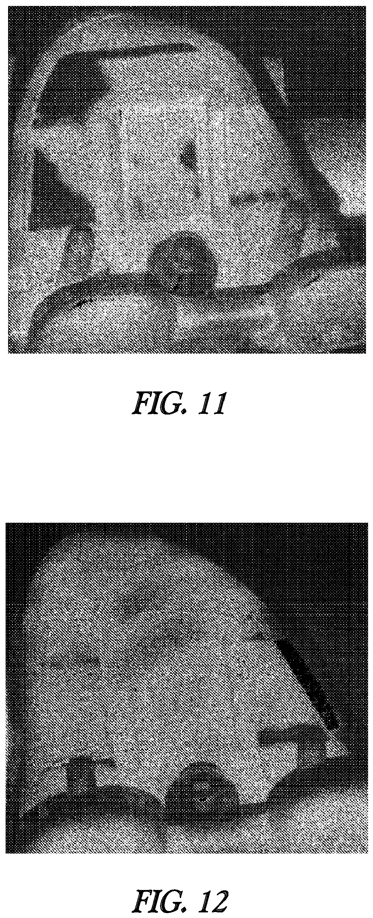

The bonding quality of each construct was then assessed by laser holography as described in for example U. S. Patent 4,408, 881. Fig. 12 shows the laser holography image for the first construct including the incompressible interlayer and Fig. 13 shows the laser holography image from the second construct including the compressible interlayer. The light grey areas indicate a quality bond between the porous tantalum structure and the implant, and the dark black areas indicate that no bond has formed between the porous tantalum structure and the implant in that particular area. As can be seen from these figures, Fig. 12 includes large areas of nonbonding and Fig. 13 includes few if any areas of nonbonding.

## Claims

1. A method of bonding, comprising:
applying a compressible interlayer, consisting essentially of a metal or a metal alloy that exhibits solid solubility with tantalum and with cobalt or cobalt-chromium, to a surface portion of a porous tantalum structure by coating the interlayer onto the surface portion of the porous tantalum structure while the structure is in a substantially flat configuration;
bending the porous tantalum structure after said surface portion has been coated with the compressible interlayer and prior to placing the exposed surface of the compressible interlayer in contact with the surface of a substrate;
positioning the compressible interlayer, between a porous tantalum structure and a substrate comprising cobalt or cobalt-chromium to form an assembly by placing an exposed surface of the compressible interlayer in contact with a surface of the substrate; and
applying heat and pressure to the assembly for a time sufficient to achieve solid-state diffusion between the substrate and the compressible interlayer and between the compressible interlayer and the porous tantalum structure.

2. The method of claim 1, wherein applying heat and pressure to the assembly includes compressing at least a portion of the compressible interlayer by collapsing one or more pores defined between interconnected metal or metal alloy particles or ligands.

3. The method of claim 2, wherein compressing the portion of the compressible interlayer includes uniformly compressing the compressible interlayer from a first thickness to a second, reduced thickness.

4. The method of any one of claims 1-3, further comprising coating the compressible interlayer on the surface portion of the porous tantalum structure without significantly occluding one or more pores of the structure.

5. The method of any one of claims 1-3, further comprising coating the compressible interlayer on the surface portion of the porous tantalum structure by plasma spraying.

6. The method of any one of claims 1-3, further comprising forming a compressible interlayer coating, having a thickness of at least about 0.025 cm (0.010 inches) prior to the application of heat and pressure, on the surface portion of the porous tantalum structure.

7. The method of any one of claims 1-6, wherein positioning the compressible interlayer between the porous tantalum structure and the substrate includes positioning at least one of hafnium, manganese, niobium, palladium, zirconium, titanium, or alloys or combinations thereof between the porous tantalum structure and the substrate.

8. The method of claim 1 wherein the compressible interlayer is formed having a substantially uniform thickness.

9. The method of any preceding claim wherein applying heat and pressure compresses the compressible interlayer.

10. The method of any preceding claim wherein after bending the porous tantalum structure, the coated surface of the tantalum structure corresponds to a geometrically complex surface of an implant..

11. The method of claim 10 wherein the geometrically complex surface of the implant includes two or more flat sections that project at an angle with respect to each other.

12. The method of claim 10 wherein said geometrically complex surface of the implant includes a rounded surface.

13. The method of any preceding claim wherein the compressible interlayer has a porosity of at least 20%.

14. The method of any preceding claim wherein the compressible interlayer is formed by plasma spraying in at least a partial vacuum so as to have a porosity between 20% and 40%.

## Patentansprüche

1. Verbindungsverfahren, umfassend:
Anbringen einer komprimierbaren Zwischenschicht, die im Wesentlichen aus einem Metall oder einer Metalllegierung besteht, das/die Festkörperlöslichkeit mit Tantal und mit Kobalt oder Kobalt- Chrom zeigt, an einen Oberflächenabschnitt einer porösen Tantal-Struktur durch Aufbringen der Zwischenschicht auf den Oberflächenabschnitt der porösen Tantal-Struktur, während sich die Struktur in einer im Wesentlichen flachen Konfiguration befindet;
Biegen der porösen Tantal-Struktur, nach dem der Oberflächenabschnitt mit der komprimierbaren Zwischenschicht beschichtet worden ist und vor dem Platzieren der exponierten Oberfläche der komprimierbaren Zwischenschicht in Kontakt mit der Oberfläche eines Substrats;
Positionieren der komprimierbaren Zwischenschicht zwischen eine poröse Tantal-Struktur und ein Substrat, das Kobalt oder Kobalt-Chrom umfasst, um eine Baugruppe zu bilden, indem eine exponierte Oberfläche der komprimierbaren Zwischenschicht mit einer Oberfläche des Substrats in Kontakt platziert wird; und
Anwenden von Wärme und Druck auf die Baugruppe für eine Zeit, die ausreichend ist, um Festkörperdiffusion zwischen dem Substrat und der komprimierbaren Zwischenschicht und zwischen der komprimierbaren Zwischenschicht und der porösen Tantal-Struktur zu erzielen.

2. Verfahren nach Anspruch 1, wobei die Anwendung von Wärme und Druck auf die Baugruppe das Komprimieren mindestens eines Abschnitts der komprimierbaren Zwischenschicht durch Kollabieren einer oder mehrerer Poren einschließt, die zwischen miteinander verbundenen Metall- oder Metalllegierungspartikeln oder Liganden definiert sind.

3. Verfahren nach Anspruch 2, wobei das Komprimieren des Abschnitts der komprimierbaren Zwischenschicht das gleichmäßige Komprimieren der komprimierbaren Zwischenschicht ab einer ersten Dicke auf eine zweite, reduzierte Dicke einschließt.

4. Verfahren nach einem der Ansprüche 1-3, das ferner das Aufbringen der komprimierbaren Zwischenschicht auf den Oberflächenabschnitt der porösen Tantal-Struktur umfasst, ohne eine oder mehrere Poren der Struktur erheblich zu okkludieren.

5. Verfahren nach einem der Ansprüche 1-3, das ferner das Aufbringen der komprimierbaren Zwischenschicht auf den Oberflächenabschnitt der Tantal-Struktur durch Plasmaspritzen umfasst.

6. Verfahren nach einem der Ansprüche 1-3, das ferner die Bildung einer komprimierbaren Zwischenschicht-Beschichtung, die eine Dicke von mindestens 0,025 cm (0,010 Zoll) vor der Anwendung von Wärme und Druck aufweist, auf dem Oberflächenabschnitt der porösen Tantal-Struktur umfasst.

7. Verfahren nach einem der Ansprüche 1-6, wobei das Positionieren der komprimierbaren Zwischenschicht zwischen der Tantal-Struktur und dem Substrat das Positionieren mindestens eines der Folgenden, nämlich von Hafnium, Mangan, Niob, Palladium, Zirconium, Titan oder Legierungen oder Kombinationen davon zwischen der porösen Tantal-Struktur und dem Substrat einschließt.

8. Verfahren nach Anspruch 1, wobei die komprimierbare Zwischenschicht mit einer im Wesentlichen gleichmäßigen Dicke geformt ist.

9. Verfahren nach einem vorhergehenden Anspruch, wobei die Anwendung von Wärme und Druck die komprimierbare Zwischenschicht komprimiert.

10. Verfahren nach einem vorhergehenden Anspruch, wobei nach dem Biegen der porösen Tantal-Struktur, die beschichtete Oberfläche der Tantal-Struktur einer geometrisch komplexen Oberfläche eines Implantats entspricht.

11. Verfahren nach Anspruch 10, wobei die geometrisch komplexe Oberfläche des Implantats zwei oder mehrere flache Abschnitte einschließt, die zueinander in einem Winkel vorstehen.

12. Verfahren nach Anspruch 10, wobei die geometrisch komplexe Oberfläche des Implantats eine abgerundete Oberfläche einschließt.

13. Verfahren nach einem vorhergehenden Anspruch, wobei die komprimierbare Zwischenschicht eine Porosität von mindestens 20% aufweist.

14. Verfahren nach einem vorhergehenden Anspruch, wobei die komprimierbare Zwischenschicht durch Plasmaspritzen in mindestens einem teilweisen Vakuum gebildet wird, um eine Porosität zwischen 20% und 40% zu haben.

## Revendications

1. Procédé de liaison comprenant :
l'application d'une intercouche compressible, constituée essentiellement d'un métal ou d'un alliage métallique qui présente une solubilité à l'état solide avec le tantale et avec le cobalt ou un mélange cobalt-chrome, sur une partie de surface d'une structure de tantale poreuse par enduction de l'intercouche sur la partie de surface de la structure de tantale poreuse alors que la structure se trouve dans une configuration pratiquement plate ;
le cintrage de la structure de tantale poreuse après enduction sur ladite partie de surface de l'intercouche compressible et avant la mise en contact de la surface exposée de l'intercouche compressible avec la surface d'un substrat ;
le positionnement de l'intercouche compressible entre une structure de tantale poreuse et un substrat comprenant du cobalt ou un mélange cobalt-chrome pour former un ensemble par mise en contact d'une surface exposée de l'intercouche compressible avec une surface du substrat ; et
l'application de chaleur et de pression sur l'ensemble pendant une durée suffisante pour obtenir une diffusion à l'état solide entre le substrat et l'intercouche compressible et entre l'intercouche compressible et la structure de tantale poreuse.

2. Procédé selon la revendication 1, ladite application de chaleur et de pression sur l'ensemble comprenant la compression d'au moins une partie de l'intercouche compressible par écrasement d'un ou plusieurs pores définis entre les particules ou ligands de métal ou d'alliage métallique interconnectés.

3. Procédé selon la revendication 2, la compression de la partie de l'intercouche compressible comprenant la compression uniforme de l'intercouche compressible d'une première épaisseur à une seconde épaisseur réduite.

4. Procédé selon l'une quelconque des revendications 1 à 3, comprenant en outre l'enduction de l'intercouche compressible sur la partie de surface de la structure de tantale poreuse sans occlusion importante d'un ou plusieurs pores de la structure.

5. Procédé selon l'une quelconque des revendications 1 à 3, comprenant en outre l'enduction de l'intercouche compressible sur la partie de surface de la structure de tantale poreuse par projection au plasma.

6. Procédé selon l'une quelconque des revendications 1 à 3, comprenant en outre la formation d'un revêtement d'intercouche compressible, ayant une épaisseur d'au moins environ 0,025 cm (0,010 pouces) avant application de chaleur et de pression, sur la partie de surface de la structure de tantale poreuse.

7. Procédé selon l'une quelconque des revendications 1 à 6, ledit positionnement de l'intercouche compressible entre la structure de tantale poreuse et le substrat comprenant le positionnement d'au moins un métal parmi le hafhium, le manganèse, le niobium, le palladium, le zirconium, le titane ou des mélanges ou combinaisons de ceux-ci entre la structure de tantale poreuse et le substrat.

8. Procédé selon la revendication 1, ladite intercouche compressible étant formée en ayant une épaisseur pratiquement uniforme.

9. Procédé selon l'une quelconque des revendications précédentes, ladite application de chaleur et de pression comprimant l'intercouche compressible.

10. Procédé selon l'une quelconque des revendications précédentes, après cintrage de ladite structure de tantale poreuse, ladite surface enduite de la structure de tantale correspondant à la surface géométriquement complexe d'un implant.

11. Procédé selon la revendication 10, ladite surface géométriquement complexe de l'implant comprenant deux sections plates ou plus qui se projettent suivant un certain angle l'une par rapport à l'autre.

12. Procédé selon la revendication 10, ladite surface géométriquement complexe de l'implant comprenant une surface arrondie.

13. Procédé selon l'une quelconque des revendications précédentes, ladite intercouche compressible présentant une porosité d'au moins 20 %.

14. Procédé selon l'une quelconque des revendications précédentes, ladite intercouche compressible étant formée par projection au plasma sous au moins un vide partiel de façon à obtenir une porosité comprise entre 20 % et 40 %.
